# EUROPÄISCHE PATENTANMELDUNG

(11) **EP 3 100 764 A1**
(43) Veröffentlichungstag der Anmeldung: **07.12.2016**
(21) Anmeldenummer: 16001127.6
(22) Anmeldetag: 18.05.2016
(51) Int. Cl.: A61N 1/36

(54) **VORRICHTUNG ZUR AUFBRINGUNG EINES TRANSKUTANEN ELEKTRISCHEN STIMULATIONSREIZES**

(30) Priorität: 03.06.2015 DE 102015007215
(71) Anmelder: cerboMed GmbH, 91052 Erlangen (DE)
(72) Erfinder: Hartlep, Andreas, 83607 Holzkirchen (DE); Frenkel, Wolf Gerhard, 72514 Inzigkofen-Engelswies (DE); Hyca, Martin, 85221 Dachau (DE)
(74) Vertreter: Gosdin, Michael

(57) **Zusammenfassung**

Die Erfindung betrifft eine Vorrichtung (1) zur Aufbringung eines transkutanen elektrischen Stimulationsreizes auf die Oberfläche eines Abschnittes des menschlichen Ohrs (2), die mindestens zwei Elektroden (3, 4) aufweist, wobei die Vorrichtung (1) eine Steuerungseinrichtung (5) umfasst, die zur Einleitung eines über die Elektroden (3, 4) fließenden Stimulationsstroms ausgebildet ist. Um die Effizienz der transkutanen Stimulation zu erhöhen, sieht die Erfindung vor, dass die Vorrichtung (1) weiterhin ein Sensorelement (6) umfasst, das mit der Steuerungseinrichtung (5) in Verbindung steht, wobei dieses Sensorelement (6) ausgebildet ist, einen ersten Parameter (Pa1) des Benutzers der Vorrichtung (1) zu erfassen, dass die Vorrichtung (1) weiterhin ein Sensorelement (6') umfasst, das mit der Steuerungseinrichtung (5) in Verbindung steht, wobei dieses Sensorelement (6) ausgebildet ist, einen zweiten Parameter (Pa2) des Benutzers der Vorrichtung (1) zu erfassen, wobei die Steuerungseinrichtung (5) ausgebildet ist, einen transkutanen elektrischen Stimulationsreiz (I) auszulösen, wenn vom Sensorelement (6, 6') ein vorgegebener Signalwert sowohl vom ersten Parameter (Pa1) als auch vom zweiten Parameter (Pa2) detektiert wird.

## Beschreibung

Die Erfindung betrifft eine Vorrichtung zur Aufbringung eines transkutanen elektrischen Stimulationsreizes auf die Oberfläche eines Abschnittes des menschlichen Ohrs, die mindestens zwei Elektroden aufweist, wobei die Vorrichtung eine Steuerungseinrichtung umfasst, die zur Einleitung eines über die Elektroden fließenden Stimulationsstroms ausgebildet ist.

Eine gattungsgemäße Vorrichtung ist aus der DE 10 2012 014 714 A1 bekannt.

Es ist generell bekannt, durch invasive und non-invasive Reizung der Nerven Einfluss auf deren neurophysiologische und neuroelektrische Qualität und damit auf die Funktion der stimulierten Nerven zu nehmen. Hierdurch können verschiedene Krankheitszustände behandelt werden. Es existieren zahlreiche Vorrichtungen sowohl zur invasiven als auch zur non-invasiven Stimulation.

Die vorliegende Erfindung stellt auf die Methode der transkutanen elektrischen Nervenstimulation ab. Bei diesem Verfahren werden Impulsströme verschiedener Stromformen, Amplituden, Impulsdauern und Frequenzen durch die Haut hindurch an verschiedenen Nerven appliziert und verändern deren Statusparameter in vorteilhafter Weise.

Eine Vorrichtung der eingangs genannten Art ist weiterhin aus der DE 10 2010 054 165 B3 bekannt. Hier ist eine Vorrichtung zur transkutanen Stimulation des Vagusnervs des menschlichen Körpers beschrieben, deren Elektrodenkopf mit zwei Elektroden im Bereich der Cymba conchae angeordnet wird; eine solche Positionierung der Elektroden hat sich als vorteilhaft erwiesen. Der Bereich der Cymba conchae ist dabei der Bereich der Concha des Ohres, der oberhalb des Crus helicis liegt; er wird auch als Hemiconcha superior bezeichnet. Unterhalb des Crus helicis nach unten erstreckt sich dann der Bereich des Cavum conchae.

Für einen zufriedenstellenden Behandlungserfolg durch Applikation eines transkutan wirkenden Stimulationsstroms ist es erforderlich, dass die Aufbringung des Stimulationsstroms nicht nur in der richtigen Stärke, sondern auch zum richtigen Zeitpunkt erfolgt.

Der vorliegenden Erfindung liegt daher die Aufgabe zugrunde, eine Vorrichtung der eingangs genannten Art bereitzustellen, mit der es möglich wird, eine verbesserte transkutane Stimulation zu ermöglichen, in dem die Vorrichtung Stimulationsimpulse automatisch zum richtigen Zeitpunkt veranlasst. Damit soll die Effizienz der transkutanen Stimulation erhöht werden können.

Die Lösung dieser Aufgabe durch die Erfindung ist dadurch gekennzeichnet, dass die Vorrichtung weiterhin ein Sensorelement umfasst, das mit der Steuerungseinrichtung in Verbindung steht, wobei dieses Sensorelement ausgebildet ist, einen ersten Parameter, insbesondere einen sich periodisch verändernden physiologischen Parameter, des Benutzers der Vorrichtung zu erfassen, dass die Vorrichtung weiterhin ein Sensorelement umfasst, das mit der Steuerungseinrichtung in Verbindung steht, wobei dieses Sensorelement ausgebildet ist, einen zweiten Parameter, insbesondere einen sich periodisch verändernden physiologischen Parameter, des Benutzers der Vorrichtung zu erfassen, wobei die Steuerungseinrichtung ausgebildet ist, einen transkutanen elektrischen Stimulationsreiz auszulösen, wenn vom Sensorelement ein vorgegebener Signalwert sowohl vom ersten Parameter als auch vom zweiten Parameter detektiert wird.

Das Sensorelement zur Erfassung des ersten Parameters und das Sensorelement zur Erfassung des zweiten Parameters können dabei durch ein einziges Sensorelement realisiert sein. Es ist genauso aber auch möglich, dass das Sensorelement zur Erfassung des ersten Parameters und das Sensorelement zur Erfassung des zweiten Parameters durch zwei separate Sensorelemente realisiert sind.

Das Sensorelement kann dabei ausgebildet sein, einen Druck, insbesondere einen Schalldruck, zu erfassen, der durch die Atmung des Benutzers der Vorrichtung erzeugt wird. In diesem Falle kann das Sensorelement ein Mikrophon sein oder ein solches aufweisen; alternativ kann das Sensorelement auch ein Drucksensor sein oder einen solchen aufweisen.

Das Sensorelement kann weiterhin auch ausgebildet sein, eine Spannung zu erfassen, die durch die Herztätigkeit des Benutzers der Vorrichtung erzeugt wird. In diesem Falle kann das Sensorelement in ein Armband, in einen Halsgurt oder in einen Brustgurt integriert sein. Weiterhin ist es möglich, dass das Sensorelement mindestens zwei Elektroden umfasst, wobei die eine der Elektroden eine derjenigen Elektroden ist, mit der die transkutane Stimulation aufgebracht ist.

Eine weitere mögliche Ausgestaltung der Erfindung sieht vor, dass das Sensorelement ausgebildet ist, die Sauerstoffsättigung des Benutzers der Vorrichtung zu erfassen, wobei das Sensorelement insbesondere ein Pulsoxymeter ist.

Eine wiederum andere Möglichkeit stellt darauf ab, dass das Sensorelement ausgebildet ist, eine Pulswelle zu erfassen, die durch die Herztätigkeit des Benutzers der Vorrichtung erzeugt wird.

Weiterhin ist es möglich, dass das Sensorelement ausgebildet ist, eine Spannung zu erfassen, die durch die Gehirntätigkeit des Benutzers der Vorrichtung erzeugt wird.

Dann sieht eine weitere Variante der Erfindung vor, dass das Sensorelement ausgebildet ist, einen Widerstand zu erfassen, die ein Abschnitt der Haut des Benutzers der Vorrichtung aufweist. In diesem Falle kann das Sensorelement in ein Armband integriert sein oder mindestens eine Klebeelektrode umfassen. Weiterhin ist es hier möglich, dass das Sensorelement die beiden Elektroden umfasst, mit denen die transkutane Stimulation aufgebracht wird.

Schließlich stellt eine weitere Variante der Erfindung darauf ab, dass das Sensorelement ausgebildet ist, eine Beschleunigung eines Körperabschnitts des Benutzers der Vorrichtung zu erfassen.

Die genannten verschiedenartigen Ausgestaltungen der Sensorelemente können dabei durchaus auch in beliebiger Kombination vorgesehen sein. Demgemäß können zwei verschiedene physiologische Parameter erfasst und der Steuerung der transkutanen Elektrostimulation zu Grunde gelegt werden.

Der Erfindung liegt die Erkenntnis zugrunde, dass es für die transkutane Neurostimulation - bezogen auf physiologische Perioden des Körpers des Benutzers der Stimulationsvorrichtung - einen jeweils optimalen Zeitpunkt gibt. Wird zu diesem Zeitpunkt, und insbesondere ausschließlich zu diesem Zeitpunkt, stimuliert, erhält man die maximale therapeutische Wirkung. Eine transkutane Stimulation über diesen optimalen Zeitpunkt hinaus, d. h. insbesondere davor oder danach, bringt dann gegebenenfalls keinen zusätzlichen Nutzen; hierdurch können die positiven Effekte sogar wieder eliminiert oder ins Gegenteil verkehrt werden.

Demgemäß sieht die Erfindung eine gezielte Auslösung (Triggerung) der Neurostimulation durch zyklische physiologische Parameter vor, d. h. namentlich die transkutane Impulsabgabe in Abhängigkeit eines bestimmten Zeitpunkts des periodischen Parameters.

Hierbei ist insbesondere an die Atmung, die Herzaktion (hierbei vor allem an das EKG, an eine Pulswelle oder an die Herzratenvariabilität (HRV) des Patienten), die Gehirntätigkeit (hierbei vor allem an das EEG, gegebenenfalls auch erfasst im Schlaf durch Auswertung der REM- bzw. NREM-Phasen), an die Hautleitfähigkeit und an den Aktivitätsgrad des Benutzers der Vorrichtung gedacht.

Für die Aufnahme der Atmungstätigkeit, insbesondere der Atemexkursionen, hat sich besonders ein Drucksensor auf dem Brustkorb bewährt, genauso kann aber auch ein Mikrofon an der Luftröhre die Atemgeräusche aufnehmen.

Für die Ermittlung des EKG bzw. der Herzratenvariabilität können beispielsweise ein Armband mit entsprechenden Elektroden, der Trage-Halsgurt des Stimulators ("Lanyard") mit eingearbeiteter Elektrode bzw. eingearbeiteten Elektroden oder Ohrelektroden vorgesehen werden; in letzterem Falle kommt auch eine für die transkutane Stimulation vorgesehene Elektrode mit einer entfernteren Gegenelektrode in Frage (die Elektrode bzw. Elektroden für die transkutane Stimulation haben dann eine Doppelfunktion, nämlich die Neurostimulation und (gemeinsam) die Aufnahme des einen Pols des EKGs). Die Gegenelektrode kann auch hier wieder im oben erwähnten Halsgurt integriert sein. Vorgesehen werden kann auch ein separater Brustgurt ("Jogging-Gurt").

Für die Erfassung einer Pulswelle oder des Sauerstoffgehalts sind Sensoren im Stand der Technik bekannt (z. B. Pulsoximeter).

Die Ermittlung des EEG kann analog zur Ermittlung des EKG erfolgen, also auch hier beispielsweise wieder mit den Elektroden, die zur Aufbringung des transkutanen Stimulationsreizes vorgesehen sind, gegebenenfalls wiederum mit einer entfernteren Gegenelektrode.

Die Hautleitfähigkeit kann beispielsweise mit einem Armband erfasst werden, in das Elektroden integriert sind, genauso kommen auch Klebesensoren bzw. Klebeelektroden in Frage.

Der Aktivitätsgrad des Benutzers der Vorrichtung kann mittels Beschleunigungssensoren erfasst werden.

Für die Datenübertragung vom genannten Sensorelement zur Steuerungsvorrichtung können verschiedene Maßnahmen eingesetzt werden (z. B. NFC (Nearfield Communication), Bluetooth oder Zigbee).

Vorgesehen werden kann auch ein Editieren der vorzunehmenden Stimulation in Abhängigkeit der vom Sensorelement ermittelten Daten. Hiernach kann eine Software vorgesehen werden, mit deren Hilfe die Stimulation punktgenau auf einen bestimmten Abschnitt der jeweiligen physiologischen Periode ausgerichtet werden kann, beispielsweise auf den Beginn der Einatmung oder auf die Q-Zacke des EKGs.

Die Art der Neurostimulation kann durch einzelne Stromimpulse oder auch durch sogenannte Bursts erfolgen.

Die Zielstruktur der Neurostimulation ist bevorzugt das vegetative Nervensystem, also der Vagus/ Parasympathikus bzw. der Sympathikus.

Bevorzugte Anwendungsgebiete der vorgeschlagenen Stimulation sind insbesondere die Behandlung von Epilepsie, von Depression und von Schmerz. Ferner kommt die Behandlung von Herzinsuffizienz, entzündlichen Erkrankungen und metabolischen Störungen in Frage.

Beispielsweise konnte beim Kipptischversuch mittels einer transkutanen Vagusnerv-Stimulation in der Aufrichtphase (Orthostase) eine Verbesserung, d. h. Erhöhung der Herzratenvariabilität, erzielt werden.

Die konkrete Umsetzung der vorgeschlagenen Maßnahmen kann durch Nutzung eines Smartphones oder iPads als Mittel zur Steuerung der Stimulation erfolgen. Hierbei können folgende Funktionen vorgesehen werden: Aufnahme und Verarbeitung der Messparameter bzw. Vitalparameter; Auswahl und Einsteuerung des optimalen Stimulations-Impulses in Richtung auf die Energie abgebende Einheit.

Es können auch die "und"-Kombination von einzelnen, oben genannten Maßnahmen vorgesehen werden, indem per Algorithmus die Maßnahmen kaskadenförmig getriggert werden ("therapeutisches Fenster"). Dabei kann die Auslösung der Stimulation auch von mehr als zwei sich überlagernden periodischen Parametern abhängig gemacht werden.

In der Zeichnung ist ein Ausführungsbeispiel der Erfindung dargestellt. Es zeigen:
- Fig. 1: schematisch die Ansicht einer Pinna (Ohrmuschel), in die eine Vorrichtung zur Aufbringung eines transkutanen Stimulationsreizes eingesetzt ist, und
- Fig. 2: schematisch den Verlauf zweier periodisch veränderlicher physiologischer Parameter über der Zeit, wobei illustriert ist, wann durch die Steuerungseinrichtung der Vorrichtung eine transkutane Elektrostimulation ausgelöst wird.

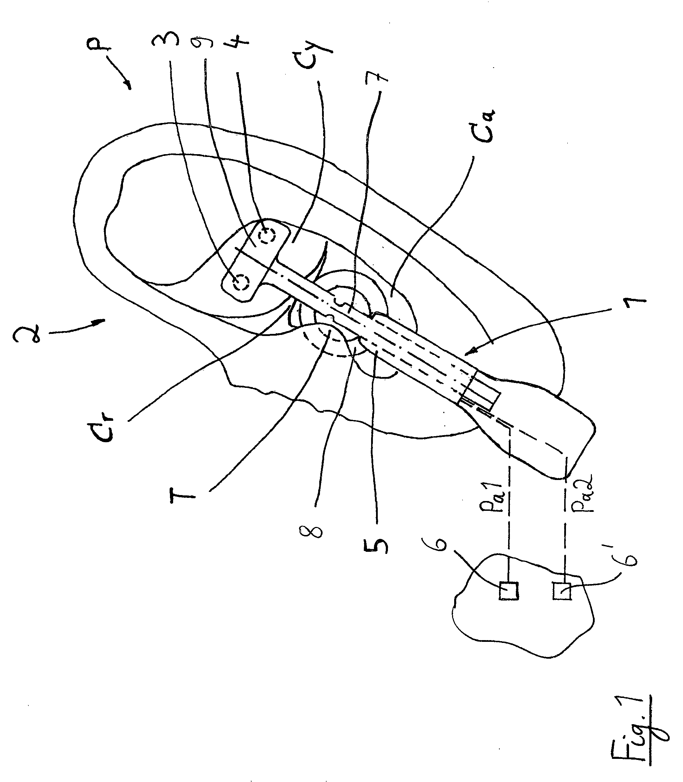

In Fig. 1 ist eine Vorrichtung 1 zur transkutanen Stimulation eines Abschnitts des menschlichen Ohrs 2 skizziert. Die Vorrichtung 1 weist eine Haltestange 7 auf, die in einem Basisteil längsverschieblich ist, das auch eine Steuerungseinrichtung 5 umfasst. Am Basisteil ist ein Auflageteil 8 angeordnet. Die generelle Positionierung der Vorrichtung 1 im Ohr 2 ergibt sich aus der Markierung der wesentlichen Bereiche des Ohrs 2, nämlich der Pinna P mit dem Cavum conchae Ca, der Cymba conchae Cy, dem Tragus T und dem Crus helicis Cr.

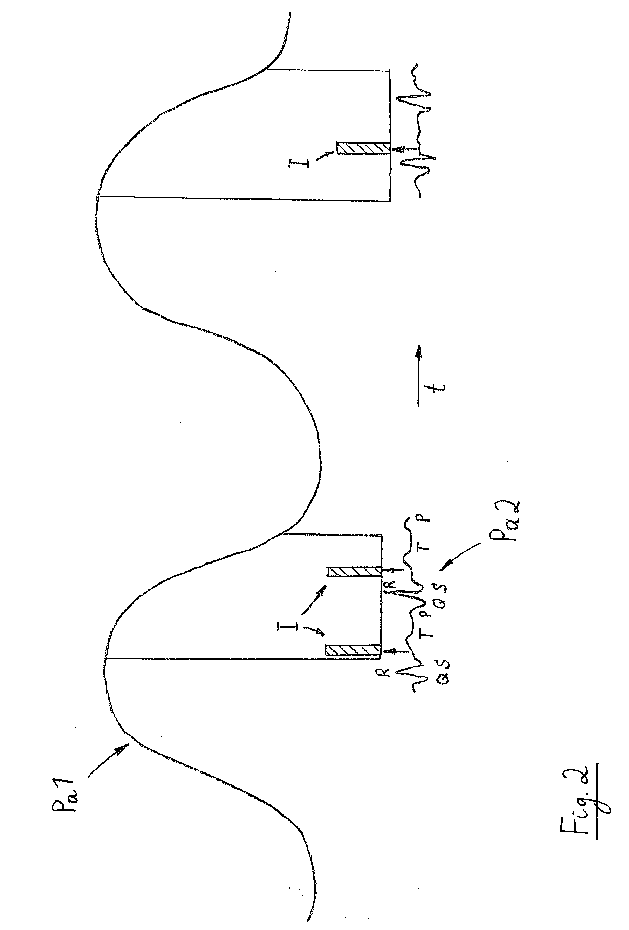

Am einen Ende der Haltestange 7 ist ein Elektrodenträger 9 angeordnet, der zwei Elektroden 3 und 4 aufweist, zwischen denen zwecks transkutaner Stimulation eine Potentialdifferenz aufgebaut wird. Hiermit wird ein Stimulationsreiz erzeugt, der in Fig. 2 mit I bezeichnet ist.

Insoweit entspricht die Vorrichtung 1 zunächst vorbekannten Lösungen, wobei insbesondere und ausdrücklich auf die DE 10 2010 054 165 B3 der Patentinhaberin Bezug genommen wird, wo eine solche Vorrichtung näher erläutert ist.

Die Vorrichtung 1 ist demnach ausgebildet, um im Bereich des Vagusnervs am Ohr 2 der die Vorrichtung benutzenden Person angebracht zu werden. Damit kann eine transkutane Stimulation des Vagusnervs vorgenommen werden.

Bestandteil der Stimulationsvorrichtung 1 ist weiterhin ein erstes Sensorelement 6 sowie ein zweites Sensorelement 6'; beide Sensorelemente 6, 6' stehen mit der Steuerungseinrichtung 5 in Verbindung. Die beiden Sensorelemente 6, 6' sind ausgebildet, jeweils einen physiologischen Parameter Pal und Pa2 zu erfassen und die gemessenen Signale der Steuerungseinrichtung 5 zuzuleiten.

Generell vorgesehen ist, dass die Steuerungseinrichtung 5 ausgebildet ist, einen transkutanen elektrischen Stimulationsreiz I (s. Fig. 2) auszulösen, wenn von den Sensorelementen 6, 6' ein vorgegebener Signalwert sowohl des ersten Parameters Pal als auch des zweiten Parameters Pa2 detektiert wird. Wie dies in einem konkreten Anwendungsbeispiel aussieht, ist in Figur 2 illustriert:
Aufgezeichnet ist hier der zeitliche Verlauf (s. Zeitachse t) eines ersten Parameters Pal sowie eines zweiten Parameters Pa2. Beim ersten Parameter Pal handelt es sich vorliegend um den Verlauf der Atmung eines Patienten, d. h. im aufsteigenden Verlauf des Parameters Pal atmet der Patient ein, während er im absteigenden Verlauf besagten Parameters ausatmet. Beim zweiten Parameter Pa2 handelt es sich vorliegend um den Verlauf der Herztätigkeit, d. h. um das EKG des Patienten (s. die typischen Punkte Q, R, S, T und P des Verlaufs).

Die Steuerungseinrichtung 5 ist im Ausführungsbeispiel nach Fig. 2 so programmiert, dass ein transkutaner Stimulationsreiz I nur veranlasst wird, wenn der per Sensorelement 6 gemessene erste Parameter Pal in der Ausatmungsphase (abfallender Bereich des detektierten Kurvenverlaufs) liegt. Eine weitere Bedingung, die kumulativ erfüllt sein muss, damit die transkutane Stimulation ausgelöst wird, ist, dass der gemessene zweite Parameter Pa2 in einem speziellen Abschnitt der gemessenen EKG-Stromkurve liegen muss. Es wird auf die Pfeile in Fig. 2 zwischen den Punkten S und T des EKG hingewiesen.

Wie nämlich in der Darstellung gemäß Fig. 2 gesehen werden kann, stellt die hier umgesetzte Lösung darauf ab, dass die transkutane Stimulation in einem therapeutischen Fenster während des Abschwungs der langsamen Periode (Parameter Pal) am besten wirkt. Innerhalb dieses Fensters bleibt genügend Zeit, die Stimulation zu einem für die schnelle Periode (Parameter Pa2) optimalen Zeitpunkt auszulösen; vorliegend ist dies - wie erläutert - jeweils zu Beginn der T-Welle des EKGs vorgesehen.

In der linken Bildhälfte gelingt demgemäß die Veranlassung eines Stimulationsreizes I zwei Mal, in der rechten Bildhälfte nur ein Mal (siehe die eingetragenen Stimulationsreize I).

### Bezugszeichenliste:

- 1: Vorrichtung zur transkutanen Stimulation
- 2: Ohr
- 3: Elektrode
- 4: Elektrode
- 5: Steuerungseinrichtung
- 6: (erstes) Sensorelement
- 6': (zweites) Sensorelement
- 7: Haltestange
- 8: Auflageteil
- 9: Elektrodenträger

- Pa1: erster Parameter
- Pa2: zweiter Parameter
- I: Stimulationsreiz

- Ca: Cavum conchae
- Cy: Cymba conchae
- T: Tragus
- Cr: Crus helicis
- P: Pinna

## Patentansprüche

1. Vorrichtung (1) zur Aufbringung eines transkutanen elektrischen Stimulationsreizes auf die Oberfläche eines Abschnittes des menschlichen Ohrs (2), die mindestens zwei Elektroden (3, 4) aufweist, wobei die Vorrichtung (1) eine Steuerungseinrichtung (5) umfasst, die zur Einleitung eines über die Elektroden (3, 4) fließenden Stimulationsstroms ausgebildet ist,
**dadurch gekennzeichnet,**
**dass** die Vorrichtung (1) weiterhin ein Sensorelement (6) umfasst, das mit der Steuerungseinrichtung (5) in Verbindung steht, wobei dieses Sensorelement (6) ausgebildet ist, einen ersten Parameter (Pa1) des Benutzers der Vorrichtung (1) zu erfassen,
**dass** die Vorrichtung (1) weiterhin ein Sensorelement (6') umfasst, das mit der Steuerungseinrichtung (5) in Verbindung steht, wobei dieses Sensorelement (6) ausgebildet ist, einen zweiten Parameter (Pa2) des Benutzers der Vorrichtung (1) zu erfassen,
wobei die Steuerungseinrichtung (5) ausgebildet ist, einen transkutanen elektrischen Stimulationsreiz (I) auszulösen, wenn vom Sensorelement (6, 6') ein vorgegebener Signalwert sowohl vom ersten Parameter (Pa1) als auch vom zweiten Parameter (Pa2) detektiert wird.

2. Vorrichtung nach Anspruch 1, **dadurch gekennzeichnet, dass** das Sensorelement (6) zur Erfassung des ersten Parameters (Pa1) und das Sensorelement (6') zur Erfassung des zweiten Parameters (Pa2) durch ein einziges Sensorelement realisiert sind.

3. Vorrichtung nach Anspruch 1, **dadurch gekennzeichnet, dass** das Sensorelement (6) zur Erfassung des ersten Parameters (Pa1) und das Sensorelement (6') zur Erfassung des zweiten Parameters (Pa2) durch zwei separate Sensorelemente realisiert sind.

4. Vorrichtung nach einem der Ansprüche 1 bis 3, **dadurch gekennzeichnet, dass** das Sensorelement (6, 6') ausgebildet ist, einen Druck zu erfassen, der durch die Atmung des Benutzers der Vorrichtung erzeugt wird.

5. Vorrichtung nach Anspruch 4, **dadurch gekennzeichnet, dass** das Sensorelement (6, 6') ein Mikrophon ist oder ein solches aufweist.

6. Vorrichtung nach Anspruch 4, **dadurch gekennzeichnet, dass** das Sensorelement (6, 6') ein Drucksensor ist oder einen solchen aufweist.

7. Vorrichtung nach einem der Ansprüche 1 bis 3, **dadurch gekennzeichnet, dass** das Sensorelement (6, 6') ausgebildet ist, eine Spannung zu erfassen, die durch die Herztätigkeit des Benutzers der Vorrichtung erzeugt wird.

8. Vorrichtung nach Anspruch 7, **dadurch gekennzeichnet, dass** das Sensorelement (6, 6') in ein Armband, in einen Halsgurt oder in einen Brustgurt integriert ist.

9. Vorrichtung nach Anspruch 7, **dadurch gekennzeichnet, dass** das Sensorelement (6, 6') mindestens zwei Elektroden umfasst, wobei die eine der Elektroden eine derjenigen Elektroden (3, 4) ist, mit der die transkutane Stimulation aufgebracht ist.

10. Vorrichtung nach einem der Ansprüche 1 bis 3, **dadurch gekennzeichnet, dass** das Sensorelement (6, 6') ausgebildet ist, die Sauerstoffsättigung des Benutzers der Vorrichtung zu erfassen, wobei das Sensorelement (6, 6') insbesondere ein Pulsoxymeter ist.

11. Vorrichtung nach einem der Ansprüche 1 bis 3, **dadurch gekennzeichnet, dass** das Sensorelement (6, 6') ausgebildet ist, eine Pulswelle zu erfassen, die durch die Herztätigkeit des Benutzers der Vorrichtung erzeugt wird.

12. Vorrichtung nach einem der Ansprüche 1 bis 3, **dadurch gekennzeichnet, dass** das Sensorelement (6, 6') ausgebildet ist, eine Spannung zu erfassen, die durch die Gehirntätigkeit des Benutzers der Vorrichtung erzeugt wird.

13. Vorrichtung nach einem der Ansprüche 1 bis 3, **dadurch gekennzeichnet, dass** das Sensorelement (6, 6') ausgebildet ist, einen Widerstand zu erfassen, die ein Abschnitt der Haut des Benutzers der Vorrichtung aufweist.

14. Vorrichtung nach einem der Ansprüche 1 bis 3, **dadurch gekennzeichnet, dass** das Sensorelement (6, 6') ausgebildet ist, eine Beschleunigung eines Körperabschnitts des Benutzers der Vorrichtung zu erfassen.

15. Vorrichtung nach Anspruch 13, **dadurch gekennzeichnet, dass** das Sensorelement (6, 6') in ein Armband integriert ist oder mindestens eine Klebeeletrode umfasst oder dass das Sensorelement (6, 6') die beiden Elektroden (3, 4) umfasst, mit denen die transkutane Stimulation aufgebracht wird.
